(19)

Europäisches
Patentamt
European
Patent Office
Office européen
des brevets

(11) **EP 2 554 123 A1**

(12) **EUROPEAN PATENT APPLICATION**
published in accordance with Art. 153(4) EPC

(43) Date of publication:
**06.02.2013 Bulletin 2013/06**

(51) Int Cl.:
*A61B 8/00* (2006.01)  *A61B 5/00* (2006.01)

(21) Application number: **11765414.5**

(22) Date of filing: **24.03.2011**

(86) International application number:
**PCT/JP2011/057134**

(87) International publication number:
**WO 2011/125513 (13.10.2011 Gazette 2011/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.03.2010 JP 2010080144**

(71) Applicant: **Hitachi Medical Corporation
Chiyoda-ku
Tokyo 101-0021 (JP)**

(72) Inventor: **CHONO, Tomoaki
Tokyo 101-0021 (JP)**

(74) Representative: **MERH-IP
Matias Erny Reichl Hoffmann
Paul-Heyse-Strasse 29
80336 München (DE)**

(54) **MEDICAL IMAGE DIAGNOSIS DEVICE, AND METHOD FOR RE-INPUTTING MEASURED VALUE OF MEDICAL IMAGE**

(57) Disclosed is a medical image diagnostic apparatus which is provided with: a measurement calculation unit configured to calculate measured values by used of multi-dimensional information relating to a target region in the medical image of an object to be examined; an image display unit configured to display the measured values and the medical image; a re-input unit for re-inputting one of the multi-dimensional information and the measured values in the medical image displayed on the image display unit; and a measurement recalculation unit configured to recalculate the other of the multi-dimensional information and the measured values, on the basis of the re-input one of the multi-dimensional information and the measured values.

FIG. 1

**Description**

Field of the Invention

The present invention relates to a medical image diagnostic apparatus and a method for re-inputting measurement values of a medical image capable of performing image diagnosis by measuring multi-dimensional information of biological tissue such as an organ in an object.

Description of Related Art

Multi-dimensional information such as a contour line in biological tissue of an object is one of the significant categories of information for evaluating the extent of a disease. In the present invention, the multi-dimensional information includes not only contour lines, but also the information on points, planes or volumes.

A medical image diagnostic apparatus is capable of noninvasively measuring a medical image of biological tissue in an object and displaying the multi-dimensional information and a medical image of the biological tissue on a display device. A medical image diagnostic apparatus includes an ultrasonic diagnostic apparatus, X-ray image diagnostic apparatus, X-ray CT apparatus, magnetic resonance imaging apparatus, and so on, and there has been a need to improve accuracy in measuring the multi-dimensional information of biological tissue in an object.

Given this factor, an image processing device is proposed in Patent Document 1 for addressing such a need of examiners. The image processing device disclosed in Patent Document 1 includes the following components (1) and (2).

(1) Contour internal area calculating means: The area inside of the contour in each image is obtained from the contour information extracted from the respective moving images. For example, the area or volume of a heart is obtained from the contour of the heart calculated using the cardiac moving images obtained by an ultrasonic diagnostic apparatus.

(2) Maximum/minimum area detecting and storing means: The maximum value, the minimum value, or both of the maximum and minimum values of the contour internal area in the moving images in a predetermined period is detected, and the internal area of the contour in the image is obtained from the detected values. For example, by detecting the cardiac cross-sectional area or the maximum value and the minimum value of the volume in moving images in a predetermined period that are obtained in (1), the area and/or the volume in the diastole and the systole can be obtained.

A series of moving images are processed by the components (1) and (2) and the contour of a cardiac wall is extracted using the moving images of the cardiac cross-sections, so as to obtain the area or volume of the cardiac region in the diastole and the systole for measuring the pumping function which is fundamental for diagnosing cardiac function.

Prior Art Documents

Patent Documents

Patent Document 1: JP-A-H10-99328

However, Patent Document 1 merely proposes a method for obtaining the area or volume of a cardiac region included in an ultrasonic image.

In reality, the positions for setting the multi-dimensional information to be extracted at the time of calculating the measurement values of an organ region vary between individuals. In order to accurately measuring the measurement values of the organ region, the examiner needs to re-input the multi-dimensional information and the measurement values in the organ region of the object in accordance with the individual difference of the object

In the above-described Patent Document 1, re-inputting performance of multi-dimensional information and measurement values of the organ region still remains as an unsolved problem.

The objective of the present invention is to provide a medical image diagnostic apparatus and the method for re-inputting measurement values of medical images capable of re-inputting multi-dimensional information and measurement values in an organ region of an object.

Brief Summary of the Invention

In order to achieve the above-described objective, the re-input unit of the present invention re-inputs one of multi-dimensional information or measurement values in a medical image displayed on the image display unit, and a measurement recalculation unit recalculates the other of the multi-dimensional information or the measurement values in conjunction with the re-input item.

In concrete terms, the medical image diagnostic apparatus related to the present invention comprising:

a measurement calculation unit configured to calculate measurement values using the multi-dimensional information of a target region in a medical image of an object; and
an image display unit configured to display the measurement values and the medical image,
further comprises:

a re-input unit configured to re-input one of the multi-dimensional information or the measurement values in the medical image displayed on the image display unit; and
a measurement recalculation unit configured to recalculate the other of the multi-dimensional information or the measurement value, on the basis of the re-input one of multi-dimensional information or the measurement value.

Also, the method for re-inputting measurement values of a medical image related to the present invention includes steps of:

displaying a medical image of an object on an image display unit;
calculating measurement values using the multi-dimensional information of a target region in the medical image;
displaying the measurement values;
re-inputting one of the multi-dimensional information or the measurement values in the medical image; and
recalculating the other of the multi-dimensional information or the measurement values on the basis of the re-input multi-dimensional information or the measurement values.

Effect of the Invention

In accordance with the present invention, the medical image diagnostic apparatus and the method for re-inputting measurement values of medical images can be provided capable of re-inputting multi-dimensional information and measurement values of an organ region in an object to be examined.

Brief Description of the Drawings

Fig. 1 is a block diagram showing the general configuration of the ultrasonic diagnostic apparatus related to the present invention.
Fig. 2 is a flowchart showing the operation procedure in a first embodiment.
Fig. 3 is a display example of a screen in the first embodiment.
Fig. 4 is a view for explaining the principle of a case using the Simpson method in calculation of re-input volume $\Delta V$.
Fig. 5 is a view for explaining an example of a method for changing a contour line.
Fig. 6 is a display example of a screen in the first embodiment which is different from that of Fig. 3.
Fig. 7 is a view for explaining the principle of calculating re-input volume $\Delta V$ using the area-length method in a second embodiment.
Fig. 8 shows the method for changing the respective axis lengths of A4C and A2C.
Fig. 9 is an example of a screen for displaying measurement result of a third embodiment.
Fig. 10 is a flowchart showing the operation procedure of a fourth embodiment.
Fig. 11 is an example of a screen for displaying measurement result of a fifth embodiment.
Fig. 12 is an example of a screen for displaying measurement result of a sixth embodiment.
Fig. 13 is an example of a screen for displaying measurement result of a seventh embodiment.
Fig. 14 is an example of a screen for displaying measurement result of an eighth embodiment.

Detailed Description of the Invention

Embodiments of the present invention will be described referring to the attached drawings.

(Description of the common part in plural embodiments of the present invention)

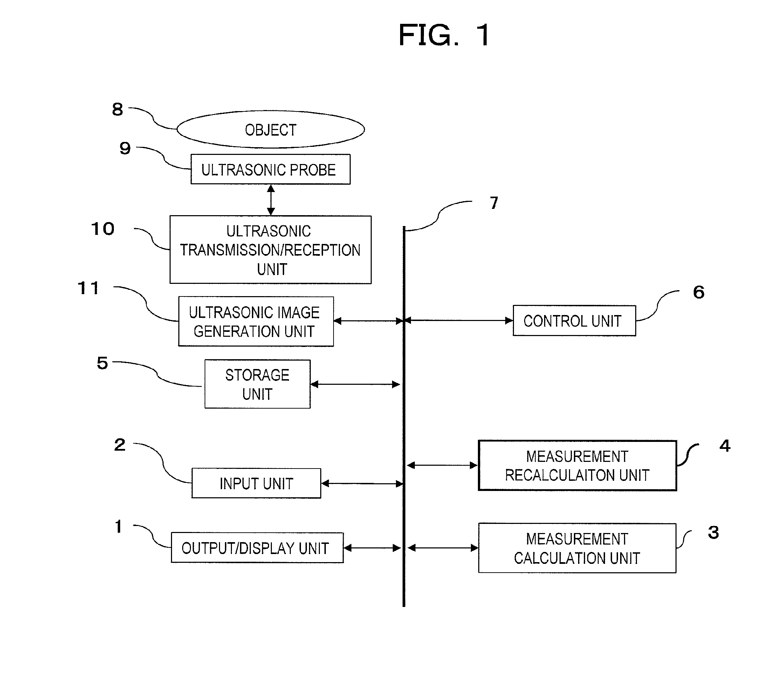

Fig. 1 is a block diagram showing the general configuration example of a medical image diagnostic apparatus related to the present invention.
The medical image diagnostic apparatus related to the present invention comprises an output/display unit 1, an input unit 2, a measurement calculation unit 3, a measurement recalculation unit 4, a storage unit 5 and a control unit 6.

The output/display unit 1 displays and outputs a medical image including a target region of an object. The output/display unit 1 displays and outputs the related information of a medical image. Concrete objects to be output or displayed by the output/display unit other than medical images are, contour lines, measurement values or a report of the measurement values. The objects are output by a video printer or as films, also by a personal computer which is connected to a network as electronic files.

The input unit 2 is an interface by which an examiner performs various operations of a diagnostic apparatus. In concrete terms, the input unit 2 sets and inputs the position of a target region displayed on the output/display unit 1. Also, the input unit 2 is an input device such as a keyboard, trackball, switch or dial, to be used for specifying the kind or feature points of biological tissue. The input unit 2 has integral configuration to share the function as a multi-dimensional information input section by which the examiner inputs multi-dimensional information of a target region and the function as a multi-dimensional information re-input section by which the examiner re-inputs multi-dimensional information of the multi-dimensional information referring to multi-dimensional information and the medical image. The multi-dimensional information input section and the multi-dimensional information re-input section in the input unit 2 may also be provided separately so that each section can function individually.

The measurement calculation unit 3 calculates the motion index of a target region using multi-dimensional information such as points, lines or regions that indicate a target region which is input by the examiner in advance using the input unit 2. The concrete examples of calculation of the motion index in a target region will be described in the first ~ eighth embodiments.

The measurement recalculation unit 4 calculates the multi-dimensional information of the motion index in the target region using the multi-dimensional information such as points, lines or regions that indicate a part of the target region which is re-input by the examiner using the input unit 2.

The storage unit 4 stores the program for operating the respective components of the output/display unit 1, the measurement calculation unit 3, the measurement recalculation unit 4, the storage unit 5 and the control unit 6 which configure the medical image diagnostic apparatus, via setting/input to the input unit 2. The storage unit 5 also stores image data.

The control unit 6 controls the respective components of the output/display unit 1, the measurement calculation unit 3, the measurement recalculation unit 4, the storage unit 5 and the control unit 6 which configure a medical image diagnostic apparatus, via setting/input to the input unit 2. A device such as a central calculation unit is used as the control unit 6.

A system bus 7 is a data transfer bus which performs mutual data communication among the connected hardware. To the system bus 7, the output/display unit 1, the input unit 2, the measurement calculation unit 3, measurement recalculation unit 4, the storage unit 5 and the control unit 6 are connected.

Also, an ultrasonic diagnostic apparatus 100 is cited in Fig. 1 as a concrete example of a medical image diagnostic apparatus. The ultrasonic diagnostic apparatus 100 further comprises an ultrasonic probe 9, an ultrasonic transmission/reception unit 10 and an ultrasonic image generation unit 11.

The ultrasonic probe 9 is applied to the body surface of an object 8 to transmit ultrasonic waves to a target region and receive the reflected echo signals which are the reflected waves of the ultrasonic waves. The ultrasonic probe 9 is provided with transducer elements of plural channels arrayed therein one-dimensionally or two dimensionally. The materials of transducer elements of the ultrasonic probe 9 are piezoelectric elements, Capacitive Micromachined Ultrasonic Transducer (CMUT), and so on.

The ultrasonic transmission/reception unit 10 provides driving signals for transmitting ultrasonic signals to the ultrasonic probe 9, receives the reflected echo signals using the ultrasonic probe 9, and performs signal processing of the received reflected echo signals. The concrete example of the signal processing of the reflected echo signals is amplification and phasing process of the reflected echo signals.

The ultrasonic image generation unit 11 converts the reflected echo signals that are processed by the ultrasonic transmission/reception unit 10 into an ultrasonic image, and outputs the ultrasonic image to the output/display unit 1.

Also, the ultrasonic transmission/reception unit 10 and the ultrasonic image generation unit 11 are connected to the control unit 6 via the system bus 7, and controlled by the control unit 6.

Next, a concrete example will be described regarding the motion index in a target region to be calculated by the measurement recalculation unit 4. One of the motion index of the target region, in the case that the target region is a heart, is an Ejection Fraction Value (abbreviated as "EF value") of the left ventricle which can be expressed by the equation (1).

$$\text{EF VALUE}\left[\%\right] = \frac{\text{DIASTOLIC VOLUME} \quad V1\left[\text{ml}\right] - \text{SYSTOLIC VOLUME} \quad V2\left[\text{ml}\right]}{\text{DIASTOLIC VOLUME} \quad V1\left[\text{ml}\right]} * 100 \qquad (1)$$

An EF value is an index to indicate the pumping function of a heart. The normal range of the EF value is about 50% and above, and the EF value decreases as the function of the heart is deteriorated.

The cardiac volume in the diastole and the systole is calculated by the control unit 6 using the following calculation method.

The volume calculation method is referred to as the Simpson method. For example, in the case that the volume of a cardiac chamber is calculated using an ultrasonic image, the Simpson method divides the volume calculation region of the cardiac chamber into a plurality of accumulated small cylindrical columns and calculates the volume of cardiac chamber as the sum of volumes of the respective small cylindrical columns, and the detailed description is disclosed in JP-A-2007-507248. Also, there are other commonly known volume calculation methods besides the Simpson method, such as the area/length method to be described in the second embodiment.

(Subject of the Invention)

In order to achieve the subject of the present invention which is to re-input measurement values or multi-dimensional information of an organ region in accordance with individual variability of the object 8, an examiner re-inputs one of the multi-dimensional information or the measurement values in a medical image displayed on the output/display unit 1 using the input unit 2, and the measurement recalculation unit 4 recalculates the other of the multi-dimensional information or the measurement values, in conjunction with the one re-input item.

(Plural Embodiments of the Present Invention)

Next, the first ~ eighth embodiments will be described.

Embodiment 1

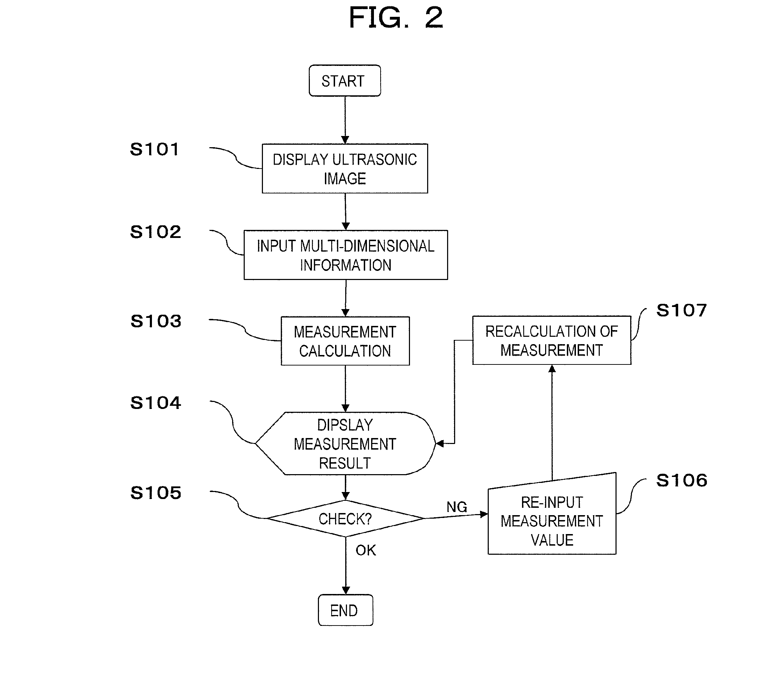

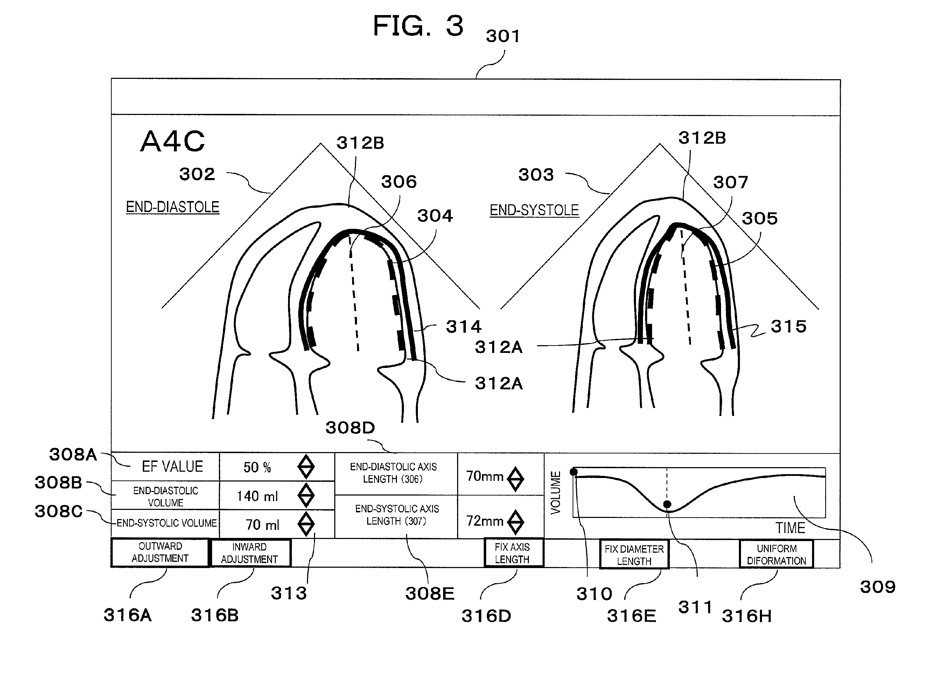

The first embodiment recalculates a contour line of the left ventricle in accordance with the re-input of an EF value and displays the EF value along with the result of the recalculated contour line, which will be described using a display example of the output/display unit 1 shown in Fig. 3 according to the processing procedure indicated in the flowchart of Fig. 2.

Fig. 2 shows the flowchart of the first embodiment, and Fig. 3 shows a display example of the output/display unit 1 in the first embodiment.

First, the control unit 6 displays, on the display screen of the output/display unit 1, an ultrasonic image in which a target biological tissue is depicted (S101).

Fig. 3 is an example that a diastolic ultrasonic image 302 and a systolic ultrasonic image 303 of the left ventricle are juxtaposed and displayed on a display screen 301, for the purpose of measurement of the left-ventricle volume and the EF value.

Next, the examiner sets the multi-dimensional information (a "contour line" here) in the input unit 2. Or, the control unit 6 may also set a contour line automatically by the automatic setting program of multi-dimensional information which is stored in the storage unit 5 (S102). As for the setting of contour lines, in the case that the target region is a heart as shown in Fig. 3, a contour line 304 in the diastole and a contour line 305 in the systole of the inner membrane of the left ventricle are set with respect to the heart.

Then the control unit 6 causes the measurement calculation unit 3 to calculate the length of the set contour lines 304 and 305 or the distance between the lines (S103).

In the case that the multi-dimensional information is not the contour lines 304 or 305 but indicated by points, the coordinate of the points or the distance among the points may also be calculated. When the multi-dimensional information is regions, the area or the volume of the regions may be calculated. As for the method of calculating areas, the method for approximating to an ellipse or rectangle can be used.

Also, the Simpson method is used in the first embodiment as the volume calculation method. The above-described calculation of contour lines is stored in the storage unit 5 as a program, to be called up at the time of measurement calculation.

Next, the control unit 6 causes the measurement values of the contour lines calculated by the measurement calculation unit 3 to be displayed on the screen of the output/display unit 7 (S104). The three display patterns of measurement values will be described below.

First Display Pattern: The concrete display pattern of measurement values calculated by the measurement calculation unit 3 performs numeric display of an EF value 308A, a diastolic volume 308B, a systolic volume 308C, a diastolic axis length 308D and a systolic axis length 308E, as shown in Fig. 3.

Second Display Pattern: The control unit 6 obtains an EF value referring to a target region on the display screen 301.

For example, the control unit 6 may also calculate an EF value using the volume of the part which is enclosed by the contour line 304 of the diastolic left-ventricle and the volume of the part which is enclosed by the contour line 305 of the systolic left-ventricle, and display the calculated value as the EF value 308A on the screen.

Third Display Pattern: The control unit 6 creates a graph of time change of a target region on the display screen 301, and obtains the EF value referring to the displayed graph. For example, the control unit 6 may calculate the left-ventricle volume in the respective frames of the ultrasonic image frame of one cardiac cycle and create a graph 309 in which the time change of the left-ventricle volume is indicated. When an EF value is calculated using the graph 309, it may be calculated by dividing a minimum value 311 of the volume by the maximum value 310 of the volume.

While the case is exemplified in which all of the first ~ third display patterns are displayed in Fig. 3, the present embodiment can be executed using at least one of the display patterns. An EF value is used as an example for displaying a measurement value.

Next, the examiner confirms whether or not the EF value on the display screen of the output/display unit 1 is adequate, and inputs "adequate (OK)" or "inadequate (NG)" to the input unit 2 (S105). The control unit 6 ends the series of measurement process when an "OK" from the input unit 2 is received. Also, the control unit 6 re-inputs the measurement value (S106) when an "NG" is inputted to the input unit 2.

The examiner properly re-inputs the EF value using the input unit 2 while confirming the EF value 308A which is displayed on the display screen 301 and the calculation process in the above-described first ~ third display patterns (S106).

The re-input of an EF value may be performed by the examiner through micro-adjusting the numeric value while pushing down a measurement value re-input switch 313 which is displayed on the screen or directly inputting the EF value using a keyboard.

The control unit 6 is provided with the function of the following examples, at the time of recalculating a contour line on the basis of the re-input EF value, to fix and control a parameter which contributes to the contour-line recalculation.

In the first example, the examiner sets the axis length as invariable by pushing down an axis length fixing switch 316D, and transforms the contour part by changing the diameter length. The axis length is the length of a segment 306 which indicates the axis that extends from a mid-point 312A to a cardiac apex 312B in the valve ring portion of the cardiac left ventricle.

In the second example, the examiner sets the diameter length of an elliptic cylinder in the Simpson method as invariable by pushing down a diameter-length fixing switch 316E, and transforms the contour part by changing the axis length.

In the third example, the examiner transforms the contour part by evenly changing the axis length or the diameter length by pushing down a uniform transforming switch 316H.

Further, besides directly re-inputting an EF value, a contour-line correcting direction specifying switch may be provided for setting a contour line on the inside or the outside of the contour in the left ventricle. In the example of Fig. 3, an inward adjustment switch 316A and an outward adjustment switch 316B are provided. The re-input result of the contour line which is moved by the inward adjustment switch 316A or the outward adjustment switch 316B is in conjunction with the measurement recalculation unit 4.

In this method, for example, when the outward switch 316B is pushed down, the contour line is adjusted outward, and the EF value 308A, the diastolic volume 308B, and the systolic volume 308C that are calculated after the adjustment of the contour line are sequentially increased and displayed. As for the method of adjusting a contour line, the moving distance or the moving method may be set in advance, for example to move the contour line outward or inward in the vertical direction by one pixel at a time, or to move the contour line in the vertical direction with respect to the segment 306.

Next, the control unit 6 causes the measurement recalculation unit 4 to calculate the moving distance of the contour line on the basis of the re-input EF value (S107).

The case that an EF value is re-input by an examiner will be described. The EF value can be calculated by the equation (1) as mentioned above. The case of re-inputting the EF value can be expressed by the following equation (2).

$$\mathrm{EF\ VALUE} + \Delta \mathrm{EF\ VALUE} = \frac{(Vd + \Delta Vd) - (Vs + \Delta Vs)}{(Vd + \Delta Vd)} * 100 \quad (\ 2\ )$$

Here, Vd is the diastolic volume and Vs is the systolic volume. Re-inputting of the EF value can be expressed by adding a desired EF value to be re-input as ΔEF value.

At this time, as shown in the right-hand side in the equation (2), diastolic volume Vd and systolic volume Vs change by the amount of re-input diastolic volume ΔVd and re-input systolic volume ΔVs. In other words, the measurement calculation unit 3 performs calculation using re-input diastolic volume ΔVd and re-input systolic volume ΔVs of the time that ΔEF value is given.

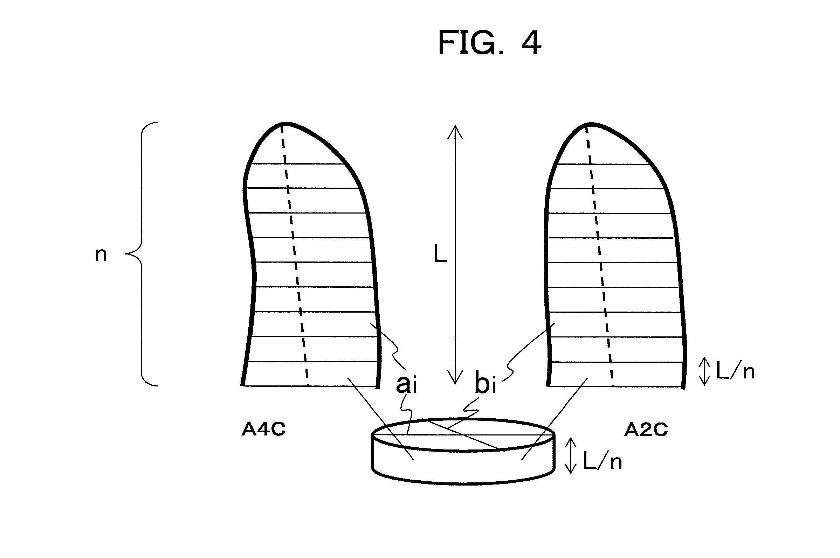

Next, the method for calculating a setting position using a volume value will be described by exemplifying the case that volume measurement is executed using the Simpson method. Fig. 4 is a view for explaining the principle of the case that the Simpson method is used for calculation of re-input volume ΔV.

An apical four-chamber image (abbreviated as "A4C image") and an apical two-chamber image (abbreviated as "A2C image") are positioned being orthogonal to each other. Further, an elliptic cylinder is assumed by setting the axis length as L, the respective distances between the wall-surfaces in the vertical directions to the axis length as a first length of the diameter (abbreviated as "first diameter length") ai and a second length of the diameter (abbreviated as "second diameter length") bi, and the distance in which the axis length is equally divided into n-numbers (n: a whole number which is greater than 1) as height L/n. Apex volume V can be calculated by obtaining each volume of n-number of elliptic cylinders and adding the obtained respective volumes, using the following equation (3). Also, the first diameter length and the second diameter length will be referred to as the diameter length.

$$V = \frac{\pi L}{4n} \sum_{i=1}^{n} \left( ai \cdot bi \right) \quad (3)$$

$$V + \Delta V = \frac{\pi \left( L + \Delta L \right)}{4n} \sum_{i=1}^{n} \left[ \left( ai + \Delta ai \right) \left( bi + \Delta bi \right) \right] \quad (4)$$

Here, the left-hand side of the equation (4) is the addition of the above-described amounts of volumetric variation ΔV. At this time, axis length L, the first diameter length ai and the second diameter length bi are changed by the portion of the re-input axis length ΔL, re-input first diameter length Δai and re-input second diameter length Δbi. In other words, re-input volume ΔV can be calculated using the re-input axis length ΔL, re-input first diameter length Δai and re-input second diameter length Δbi.

Next, a method for setting the following conditions will be described with respect to re-input volume ΔV.

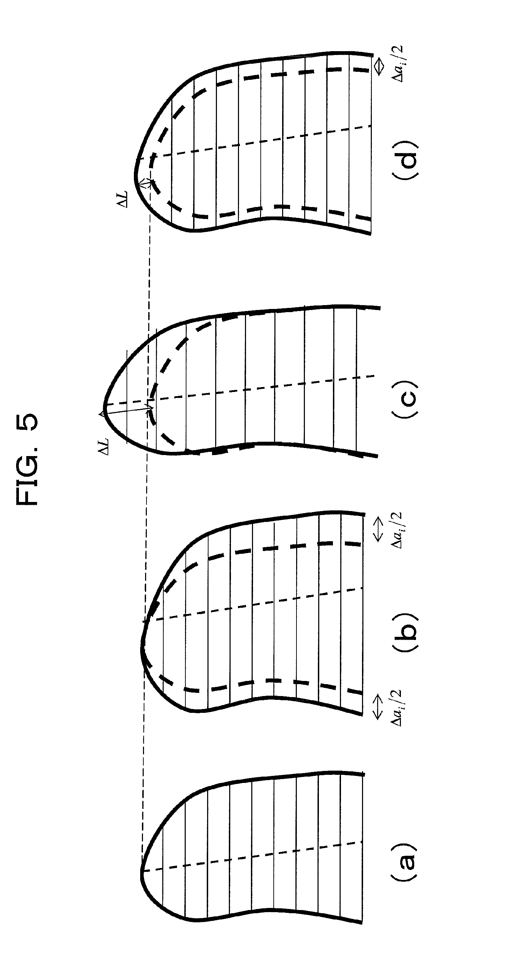

Fig. 5 is a view for explaining the method for changing the position of a contour line.

Fig. 5(a) indicates a contour line before the change.

In Fig. 5(b), the dotted line indicates the contour line before the change, and the solid line indicates the contour line after the change. The operation before and after the examiner changes the contour line as shown in Fig. 5(b) is to first push down the axis length fixing switch 316D to fix axis length L, i.e. to set re-input axis length ΔL=0. The control unit 6 changes the first diameter length ai to ai+Δai, and the second diameter length bi to bi+Δbi. Here, Fig. 5(b) is approximated to a circular form setting as Δai=Δbi.

Fig. 5(b) is useful as a method for re-inputting a contour line which places significance on an error in the axis length. The method shown in Fig. 5(b) is sufficient for re-inputting a contour line, since volume measurement of a heart chamber by the Simpson method promulgates to keep the error in the axis length of A4C and the axis length of A2C within 10%. In Fig. 5(c), the broken line indicates the contour line before the change, and the solid line indicates the contour line after the change, as in Fig. 5(b). The operation before and after the examiner changes the contour line as shown in Fig. 5(c) is to first push down the diameter-length fixing switch 316E in Fig. 3 to fix first diameter length ai and second diameter length bi, i.e. to set re-input first diameter length Δai=0 and re-input second diameter length Δbi=0. The control unit 6 calculates ΔL using the equation (4) by changing only axis length L to L+ΔL and setting Δai=0 and Δbi=0. The change of axis length to L+ΔL is performed assuming that height L/N of the respective elliptic cylinders is evenly changed.

Fig. 5(c) is useful as a method for re-inputting a contour line which places significance on an error in the diameter length. Cardiac motion of an object varies among individuals and coping with the error in the axis length is usually sufficient for re-inputting a contour line in many cases. However, the method for re-inputting a contour line shown in Fig. 5(c) is effective for measurement of those who need measures to cope with the error in the diameter direction.

In Fig. 5(d), the broken line indicates the contour line before the change and the solid line indicates the contour line after the change, as in Fig. 5(b). The operation before and after the examiner changes the contour line as shown in Fig. 5 (d) is to first push down the uniform transforming switch 316H in Fig. 3. The control unit 6 changes all of axis length L, first diameter-length ai and second diameter-length bi. In Fig. 5(d), the equation (4) is calculated assuming that the respective values of re-input axis length ΔL, a half of re-input first diameter length (Δai/2) and a half of re-input second diameter length (Δbi/2) are equal. As shown in Fig. 5(d), the axis length and the diameter length are almost equally changed.

Fig. 5 (d) is useful as a method for re-inputting a contour line which places significance on an error in the axis length and the diameter length. In the case that re-input of a contour line is difficult even with consideration of the error in the axis length (Fig. 5(b)) or the error in the diameter length (Fig. 5(c)) due to individual difference in cardiac motion of an object, Fig. 5(d) is sufficient as the method for re-inputting a contour line by making the errors in the axis length and the diameter length equal.

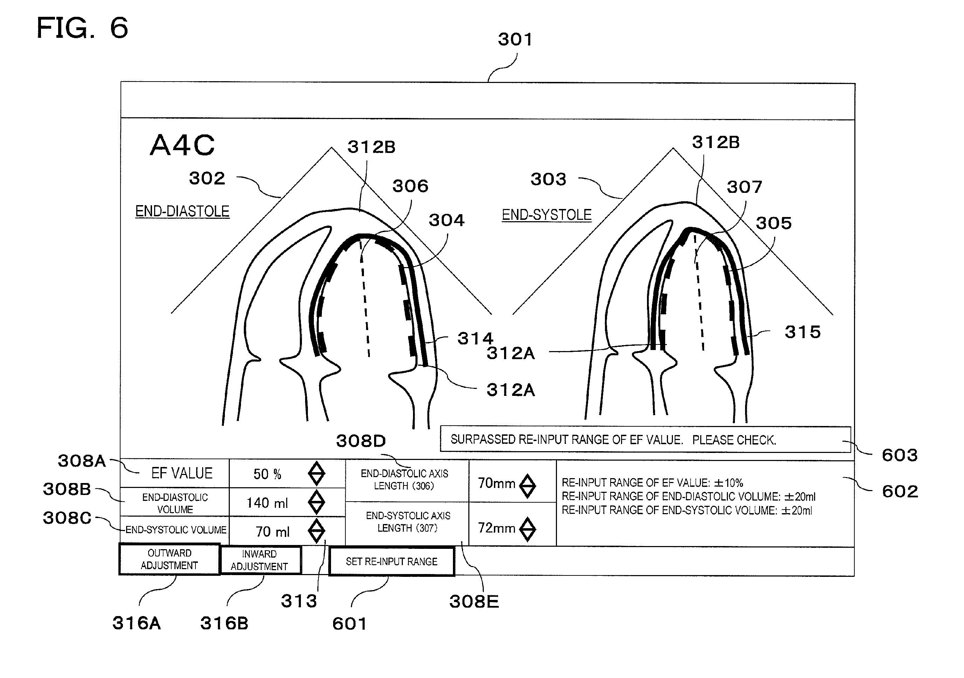

Also at the time of changing a contour line, the upper limit value and the lower limit value of a re-input amount of EF value 308A, etc. may also be set by pushing down the re-input range setting switch 601 in Fig. 6.

Fig. 6 is a display example which is different from the screen of the first embodiment shown in Fig. 3. The re-input range is set in advance, for example, as re-input amount of EF value 308A: within $\pm10\%$, and re-input amount of diastolic volume 308B and systolic volume 308C: within $\pm20ml$, as shown in the display region 602.

The control unit 6 implements warning display on the output/display unit 1 when the re-input amount surpasses the preset re-input range.

Warning display is to be implemented, for example by displaying a warning sentence such as "Message (abbreviated as "MSG" in the diagram): Surpassed re-input range of EF value. Please confirm" or coloring the numeric value of the EF value 308A when the value reaches the limit value, as shown in the display range 602 of the screen 301 in the output/display unit 1.

The example shown in Fig. 6 is useful for reminding the examiner when re-input operation of a contour line is performed which surpasses the allowed re-input range.

Further, in the case that the examiner needs to re-input a contour line, S106, S107, S103, S104 and S105 can be repeated until re-input operation is no longer necessary.

In accordance with the above-described first embodiment, it is possible to provide the medical image diagnostic apparatus and the method for re-inputting measurement values of a medical image capable of re-inputting multi-dimensional information and measurement values of an organ region in an object. Also, characteristic advantage of the first embodiment is, in volume calculation, that an examiner can arbitrarily choose whether or not to adjust the axis length and/or the diameter length of a heart chamber using the Simpson method, which enables the multi-dimensional information (contour line) to be partially changed.

Embodiment 2

In the second embodiment, the area/length method is used in place of the Simpson method which is used in the first embodiment, in calculation of re-inputting multi-dimensional information (S107) in the first embodiment.

Only the calculation method in S107 will be described below which is the part that is different from the first embodiment.

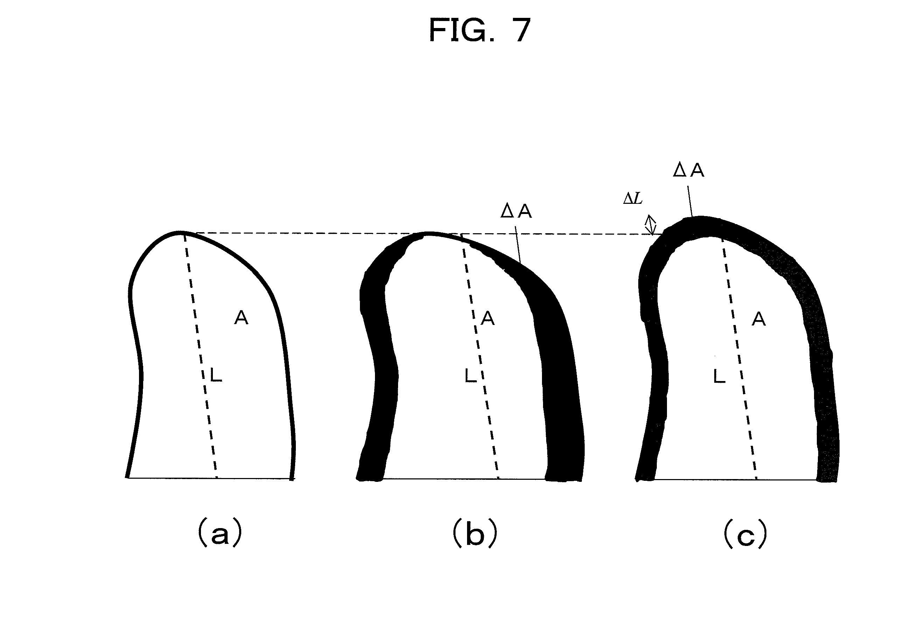

Fig. 7 is a view for explaining the calculation principle of re-input volume $\Delta V$ using the area/length method in the second embodiment. The area/length method calculates volume V of the left ventricle by the equation (5) using the area A of the region framed by the contour lines 304 and 305 and axis length L which extends from the mid-point between the valve ring portions 312 to the cardiac apex shown in Fig. 3.

Also, addition of volume V and volume variation $\Delta V$ in the left ventricle can be expressed by the following equation (6).

$$V = \frac{8A^2}{3\pi L} \quad (5)$$

$$V + \Delta V = \frac{8(A + \Delta A)^2}{3\pi(L + \Delta L)} \quad (6)$$

At this time, re-input portions of area A and axis length L are set as re-input area ΔA and re-input axis length ΔL respectively. As in the first embodiment, ΔV is calculated using re-input area ΔA and re-input axis length ΔL.

Next, the method for setting the above-mentioned conditions will be described. Fig. 7 shows the examples in which the positions of the contour lines 304 and 305 are changed by setting conditions.

Fig. 7(a) shows the contour line before being changed.
Fig. 7(b) is an example that the axis length is fixed using the axis-length fixing switch 316D. This is the method that changes only area A by fixing the axis length L. In other words, re-input area ΔA is calculated using the equation (6) by setting re-input axis length AL=0. Area A is to be calculated by moving the contour line 304 or the contour line 305 without changing axis length L as shown in Fig. 7(b).

Fig. 7(c) is an example that the area and the axis length are made variable. This case is to be selected in the case that a switch such as the axis-length fixing switch 316D is not pushed down. The control unit 6 schematically displays Fig. 7(c) on a part of display screen 301, and arbitrarily sets re-input area ΔA and re-input axis length ΔL by the operation of the input unit 2 (for example, a mouse). Re-input volume ΔV is calculated by applying the set re-input area ΔA and re-input axis length ΔL to the equation (6) (S107).

In accordance with the above-described second embodiment, it is possible to provide the medical image diagnostic apparatus and the method for re-inputting measurement values capable of re-inputting the multi-dimensional information and the measurement values of an organ region in an object. Also, the characteristic advantage of the second embodiment is that an examiner can arbitrarily select whether or not to adjust the axis length and/or the area for volume calculation by the area/length method, in order to partially change the multi-dimensional information (contour line).

Embodiment 3

The third embodiment, in the calculation of re-input multi-dimensional information (S107) of the first embodiment, uses a method which is different from the Simpson method used in the first embodiment, for calculating the axis length. Only the calculation method in S107 will be described below which is the different part from the first embodiment.

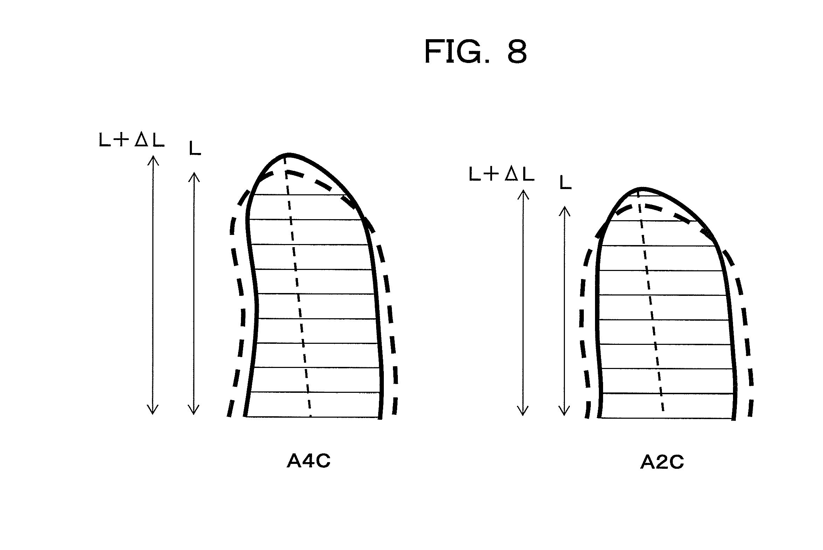

Fig. 8 shows the method for changing the respective axis lengths of A4C and A2C. In measurement of EF values, it is generally promulgated to make the error of the axis lengths of A4C and A2c within 10%. Thus after the result of axis-length measurement is displayed as shown in Fig. 3, there are cases that these axis lengths are re-input to make them within the promulgated error range.

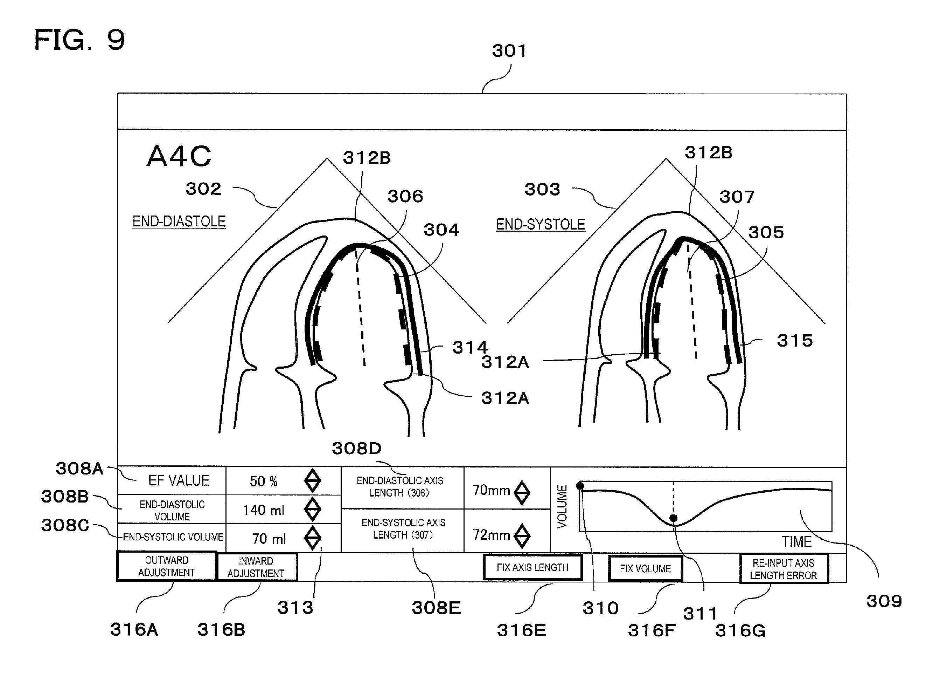

The examiner may re-input the information by fine-adjusting the numeric values by pushing down the axis-length error re-input switch 316G as shown in Fig. 9 using a mouse which is provided to the input unit 2, or may directly input the EF value using the keyboard which is provided to the input unit 2. Fig. 9 is an example of a screen in which the measurement result of the third embodiment is displayed. The control unit 6 re-inputs axis length L as L+ΔL using the input unit 2 upon receiving the input value.

At this time, if the examiner selects whether to fix the first diameter length and the second diameter length using the diameter-length fixing switch 316E or to fix the volume by the volume fixing switch 316G in advance, the equation (4) or the equation (6) can be performed with the selected condition. The example in Fig. 8 indicates the contour lines in which the axis length is changed by the portion of ΔL in the condition that the volume is fixed. The broken lines indicate the contour lines before the change, and the solid lines indicate the contour lines after the change.

Also, the above-mentioned axis-length error re-input switch 316G may also control the measurement recalculation unit 4 and measurement calculation unit 3 so that the control unit 6 automatically adjusts ΔL to make the axis-length error of the A4C and A2C within 10% by merely receiving the input by the examiner via the input unit 2 (S107).

In accordance with the above-described third embodiment, it is possible to provide the medical image diagnostic apparatus and the method for re-inputting measurement values of a medical image capable of re-inputting multi-dimensional information and measurement values of an organ region in an object. Also, characteristic advantage of the third embodiment is, in an EF value measurement, that the difference between the axis lengths A4C and A2C can be automatically corrected so that the labor of manual re-input operation by an examiner can be reduced.

Embodiment 4

In the example of the fourth embodiment, an examiner inputs a re-input EF value in a display column of the EF value 308A using the input unit 2, and sets a contour line to be superimposed and displayed over an ultrasonic image in accordance with the input EF value.

The fourth embodiment is different from the first embodiment in that setting of measurement values (S201) is inserted between display of ultrasonic image of biological tissue (S101) and setting of multi-dimensional information (S102). Only the different part will be described below since the rest is the same as the first embodiment.

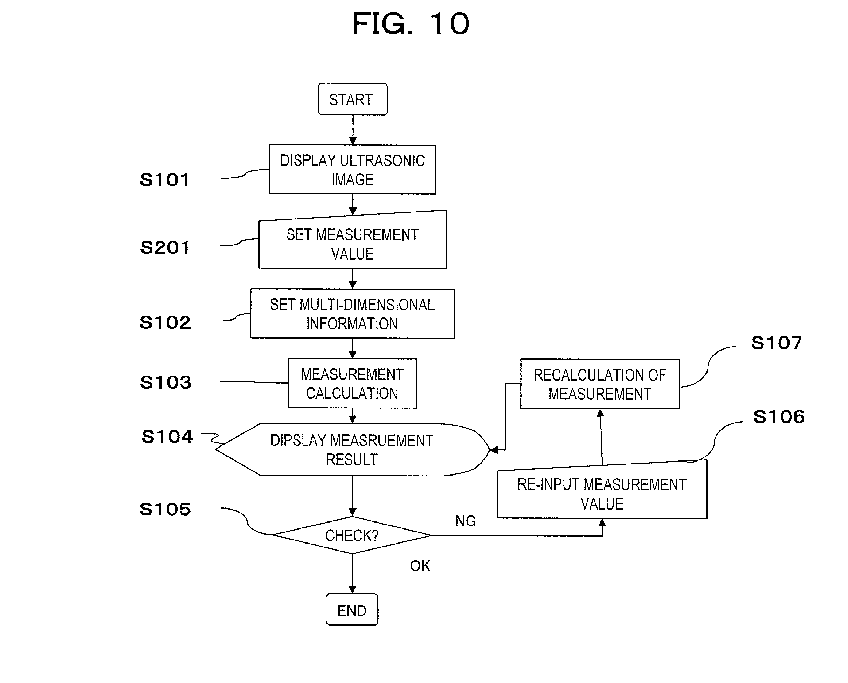

The procedure will be described using the flowchart of Fig. 10. Fig. 10 is a flowchart showing the operation procedure of the fourth embodiment. The examiner sets a desired EF value in an EF value list 308A in Fig. 3 using the input unit 2 while observing the ultrasonic image displayed in S101 (S201).

The control unit 6 calculates re-input multi-dimensional information (S107) so as to calculate the EF value which is set in S201. Then the measurement setting position is displayed on the screen (S104). After confirming the result (S105), the examiner re-inputs the EF value if necessary by operating the input unit 2 (S106).

In accordance with the above-described fourth embodiment, it is possible to provide the medical image diagnostic apparatus and the method for re-inputting measurement values of a medical image capable of re-inputting multi-dimensional information and measurement values of an organ region in an object. Also, characteristic advantage of the fourth embodiment is, when an EF value can be predicted in advance, that the predicted EF value can be first set before setting the multi-dimensional information (contour line).

Embodiment 5

The fifth embodiment is a method, in measurement result display (S104) of the first embodiment, which presents plural candidates of a re-input contour line so that an examiner can select a contour line from among the presented plural candidates. Only the difference from the first embodiment will be described below, which is the measurement result display (S104) regarding the re-input multi-dimensional information and the screen display.

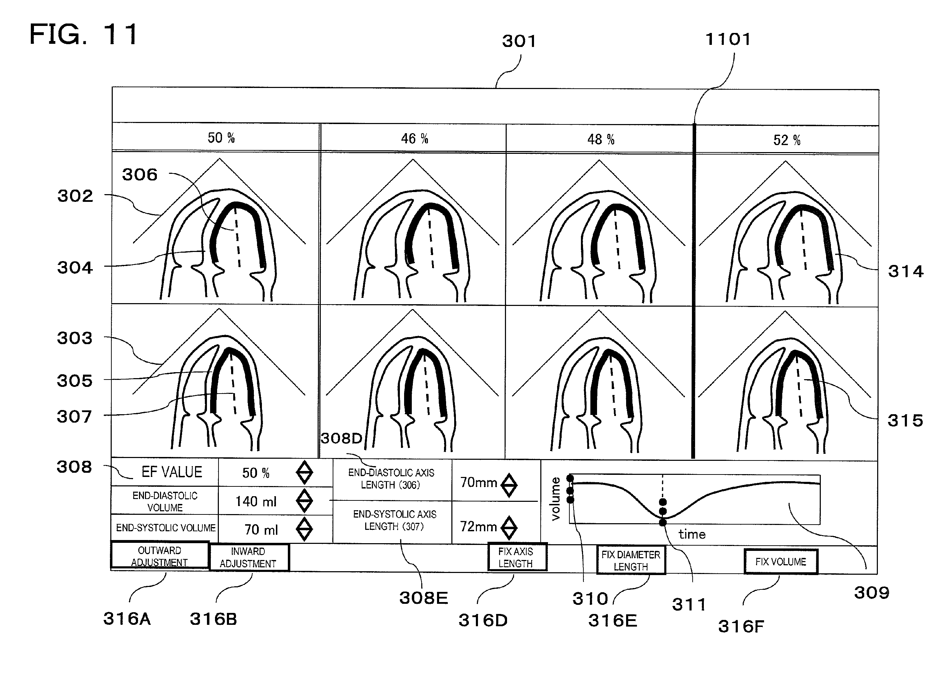

Fig. 11 is an example of the screen in which measurement result of the fifth embodiment is displayed. In the screen of Fig. 11, a contour line with 50% of EF value is displayed on the left row. On the right side thereof, ultrasonic images of the contour lines with 46%, 48% and 52% of EF values, which are around 50% of EF values, are juxtaposed in order. Then a part in which the ultrasonic image with 50% of EF value is to be inserted is indicated with a reference numeral 1101.

After an EF value is re-input as 50% (S103), the values are set in which the EF value is changed to be re-input around 50% (S106). While the examples of other EF values around 50% are changed by 2% here, the variation of re-input EF values can be changed by an arbitrary amount.

Next, the respective contour lines are calculated by calculating the re-input multi-dimensional information on the basis of the inputting EF value (S107). Here, the respective contour lines are calculated with EF values of 46%, 48% and 52%.

Fig. 11 is a screen on which contour lines with respect to plural EF values are displayed. The ultrasonic images in the upper part indicate the images in diastole, and the ultrasonic images in the lower part indicate the images in systole. The examiner selects and determines the optimum contour line while checking the contour lines on the respective displayed ultrasonic images (S105).

In this manner, in the case that a disease is suspected when the EF value is less than 50% and the normal range of the EF value is above 50%, the examiner can confirm whether the cardiac motion is in the normal range or a disease is suspected by making a comprehensive evaluation of cardiac movement in diastole and systole.

In accordance with the above-described fifth embodiment, it is possible to provide the medical image diagnostic apparatus and the method for re-inputting measurement values of a medical image capable of re-inputting multi-dimensional information and measurement values of an organ region in an object. The characteristic advantage of the fifth embodiment is that diagnostic information made by a conventional evaluation of the cardiac motion in the diastole and the systole can be provided. Also, the examiner can select the optimum multi-dimensional information (contour line) and EF value while checking the change of multi-dimensional information (contour line) with respect to the minute variation of the EF value.

Embodiment 6

The sixth embodiment is a modification of measurement result display (S104) in the fifth embodiment, which displays the A4c-image referring to the A2C-image. Only the difference from the fifth embodiment will be described below, which is the measurement result display (S104) regarding the re-input of contour line and the screen display.

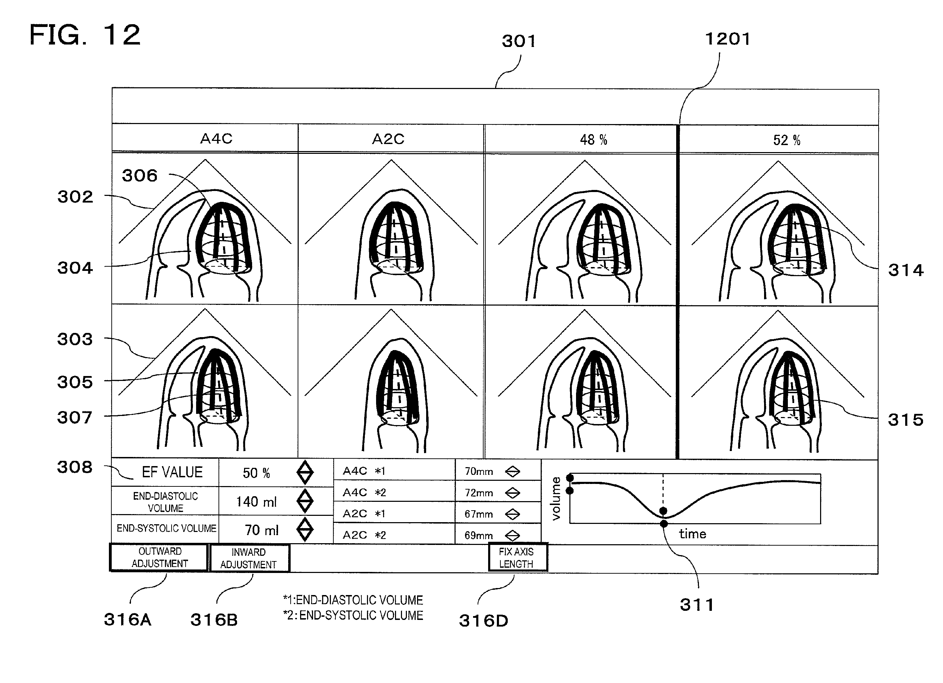

Fig. 12 shows an example of the screen in which measurement result of the sixth embodiment is displayed. The ultrasonic image 302 and the ultrasonic image 303 on the far-left side in Fig. 12 are the A4C-images in which the EF value is set as 50%. The images displayed on the immediate right to the ultrasonic 302 and the ultrasonic image 303 are the A2C-images. The upper images of the A4C-images and the A2C-images are of the end-diastole and the lower ultrasonic images thereof are of the end-systole.

Since the A4C-images and the A2C-images are positioned orthogonal to each other, the elliptic cylinders accumulated using each other's diameter are 3-dimensionally displayed.

In the two rows on the right side of the A2C-images, the A4C-images of 48% and 52% with 50% of the EF value are displayed.

While A4C-images are displayed in Fig. 12, A2C-images may also be displayed. The examiner selects and determines the optimum contour line while checking the 3-dimensionally displayed contour lines (S105).

In accordance with the above-described sixth embodiment, it is possible to provide the medical image diagnostic apparatus and the method for re-inputting measurement values of a medical image capable of re-inputting multi-dimensional information and measurement values of an organ region in an object. Also, characteristic advantage of the sixth embodiment is, since A4C-images and A2C-images are the ultrasonic images showing positional relationship that are orthogonal to each other, that re-input of multi-dimensional information (contour line) can be executed on the basis of the 3-dimensional information.

Embodiment 7

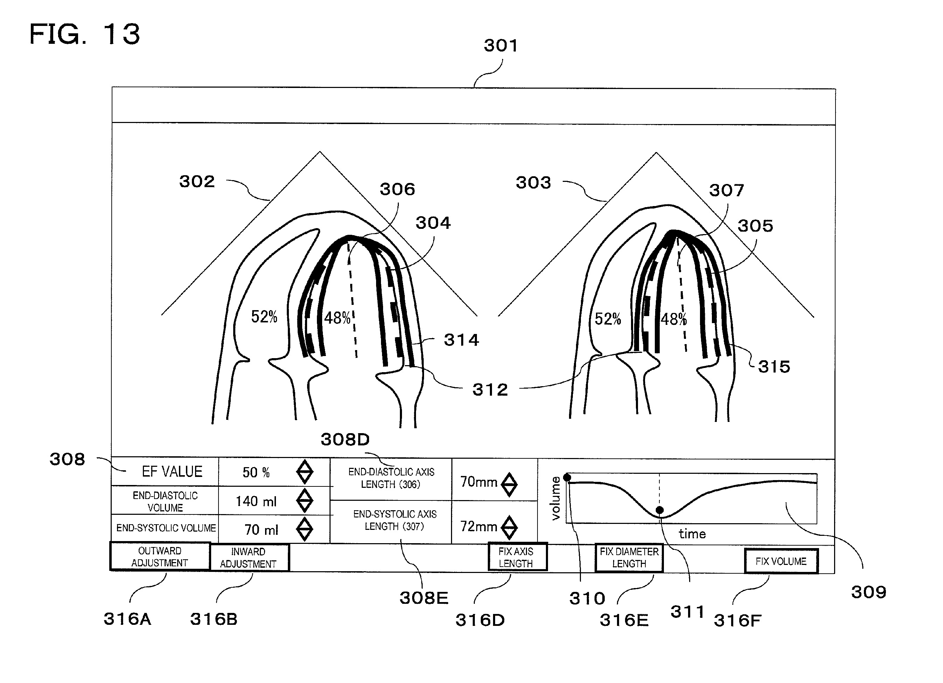

The seventh embodiment, in measurement result display (S104) of the first embodiment, is that plural candidates of re-input contour lines are displayed on the same ultrasonic image, and an examiner can select a contour line from among the displayed plural candidates. Only the difference from the first embodiment will be described below, which is the measurement result display (S104) regarding the re-input of contour line and the screen display. Fig. 13 is an example of the screen in which plural measurement results of the seventh embodiment are displayed.

Broken lines (304 and 305) indicate the contour lines with the EF value of 50%. On the outside of the broken contour lines, the contour lines with the EF value of 52% are indicated with solid lines.

Further, on the inside of the contour lines with 50% of EF value, the contour lines with 48% of EF value are indicated with solid lines. The examiner can select and determine the optimum contour line while checking the plural contour line candidates displayed at the same time (S105). Though the kind of lines is changed to display contour lines in the seventh embodiment, any display pattern may be used to differentiate plural contour lines such as changing colors or blinking different lines.

Also, while 2-dimensional contour lines are exemplified in the seventh embodiment referring to Fig. 13, 3-dimensional contour lines as in the sixth embodiment may also be displayed.

In accordance with the above-described seventh embodiment, it is possible to provide the medical image diagnostic apparatus and the method for re-inputting measurement values of a medical image capable of re-inputting multi-dimensional information and measurement values of an organ region in an object. Also, characteristic advantage of the seventh embodiment is, since different contour lines are displayed by one ultrasonic image and only two image displays of the diastole and the systole are necessary, multi-dimensional information (contour lines) can be observed with less display screens compared to the plural groups of screen display such as the fifth embodiment.

Embodiment 8

The eighth embodiment, in the measurement result display (S104) of the first embodiment, is the method that the contour line in healthy condition at the present time is displayed so that healing progression can be observed. The only difference from the first embodiment will be described in the eighth embodiment, which is the measurement result display (S104) regarding the input of contour lines and the screen display.

For example, in dilated cardiomyopathy, the systolic volume increases and the EF value decreases. Given this factor, a treatment to decrease the systolic volume and to increase the EF value is often carried out by drug administration. At this time, the contour line with the normal volume and EF value can be calculated using the present contour line and displayed on the screen, which helps the examiner to visually recognize the difference between the present cardiac condition and the healthy cardiac condition.

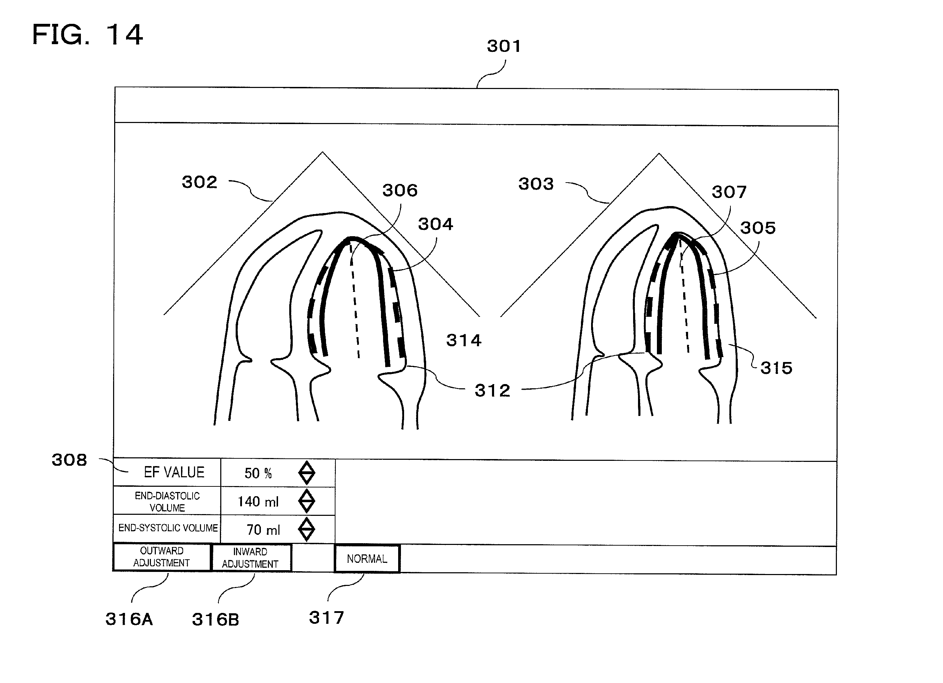

Fig. 14 is an example of a screen in which plural measurement results in the eighth embodiment are displayed. Fig. 14 shows a contour line (broken line) with respect to the EF value of a disease and a contour line (solid line) with respect to the normal EF value. By pushing down a normal-example setting switch 317 after setting a contour line with respect to the EF value of a disease, a solid contour line with respect to the EF value of a disease is displayed.

The standard values of normal volume or EF value are stored in the storage unit 5 in advance. The control unit 6 reads out the standard value and calculates a contour line of the standard value. A contour line of the standard value, for example, should be the average of multiple examples of a normal value or the target value of a patient.

The control unit 6 may also store in the storage unit 5 the value which is measured in an examination at least one time in the past, so as to superimpose and display these data on the screen in the present examination. In this manner, continuous treatment process from the past examination to the present one can be visually recognized.

In accordance with the above-described eighth embodiment, it is possible to provide the medical image diagnostic apparatus and the method for re-inputting measurement values of a medical image capable of re-inputting multi-dimensional information and measurement values of an organ region in an object. Also, characteristic advantage of the eighth embodiment is that continuous treatment process can be visually recognized from the past examination to the present one.

While cardiac measurement using an ultrasonic diagnostic apparatus is exemplified in the above embodiments, the present invention can be applied also to other apparatuses and biological tissue.

Description of Reference Numerals

1: output/display unit
2: input unit
3: measurement calculation unit
4: measurement recalculation unit

**Claims**

1.  A medical image diagnostic apparatus comprising:

    a measurement calculation unit configured to calculate a measurement value using multi-dimensional information of a target region in a medical image of an object to be examined; and
    an image display unit configured to display the measurement value and the medical image,
    further comprising:

        a re-input unit configured to re-input one of the multi-dimensional information or the measurement value in the medical image displayed on the image display unit; and
        a measurement recalculation unit configured to recalculate the other of the multi-dimensional information or the measurement value, on the basis of the re-input one of multi-dimensional information or the measurement value,.

2.  The medical image diagnostic apparatus according to claim 1, wherein the re-input unit has an input unit configured to input the multi-dimensional information.

3.  The medical image diagnostic apparatus according to claim 1, wherein the re-input unit inputs numerical values of the measurement value.

4.  The medical image diagnostic apparatus according to claim 1, wherein the re-input unit inputs the multi-dimensional information on the medical image in which the target region is displayed.

5.  The medical image diagnostic apparatus according to claim 1, wherein measurement recalculation unit calculates one of the multi-dimensional information or the measurement value on the basis of the motion index of the target region.

6.  The medical image diagnostic apparatus according to claim 1, wherein the measurement recalculation unit generates a graph of the time change in an area of the target region and displays the graph on the image display unit.

7.  The medical image diagnostic apparatus according to claim 1, wherein the measurement recalculation unit displays on the image display unit a warning about the re-input operation which surpasses an allowed re-input range.

8.  The medical image diagnostic apparatus according to claim 1, wherein:

    the measurement value is the volume of the target region; and
    the measurement recalculation unit calculates the multi-dimensional information or the measurement value using the Simpson method or the area/length method for calculating the volume of the target region.

9.  The medical image diagnostic apparatus according to claim 1, wherein:

    the measurement value is the length of the target region; and
    the measurement recalculation unit calculates the multi-dimensional information or the measurement value based on the axis length of the orthogonal cross-section in the target region.

10. The medical image diagnostic apparatus according to claim 1, wherein an input is executed to a display column of the measurement value using the re-input unit, and the remeasurment calculation unit calculates the multi-dimensional information.

11. The medical image diagnostic apparatus according to claim 1, wherein the measurement recalculation unit calculates the plural measurement values and the plural sets of multi-dimensional information, and displays groups of the plural measurement values, multi-dimensional information and the medical images on the medical display unit.

12. The medical image diagnostic apparatus according to claim 11, wherein the measurement recalculation unit uses the orthogonal cross-sections in the plural sets of multi-dimensional information.

13. The medical image diagnostic apparatus according to claim 1, wherein the measurement recalculation unit calculates the plural sets of multi-dimensional information, superimposes and displays the plural sets of multi-dimensional information and the medical image on the image display unit.

14. The medical image diagnostic apparatus according to claim 1, wherein the measurement recalculation unit calculates the multi-dimensional information of normal condition, superimposes and displays the multi-dimensional information of normal condition and the medical image on the image display unit.

15. A method for re-inputting a measurement value of medical image including steps of:

displaying a medical image of an object on an image display unit;
calculating a measurement value using the multi-dimensional information of a target region in the medical image;
displaying the measurement value;
re-inputting one of the multi-dimensional information or the measurement value in the medical image; and
recalculating, on the basis of the re-input multi-dimensional information or the measurement value, the other of the multi-dimensional information or the measurement value.

# FIG. 1

# FIG. 2

START

S101 — DISPLAY ULTRASONIC IMAGE

S102 — INPUT MULTI-DIMENSIONAL INFORMATION

S103 — MEASUREMENT CALCULATION

S107 — RECALCULATION OF MEASUREMENT

S104 — DIPSLAY MEASUREMENT RESULT

S105 — CHECK?

NG

S106 — RE-INPUT MEASUREMENT VALUE

OK

END

# FIG. 3

**301**

**A4C**

**302** END-DIASTOLE

**303** END-SYSTOLE

312B, 306, 304, 314, 312A, 312A, 308D

312B, 307, 305, 315

| | | | | | |
|---|---|---|---|---|---|
| **308A** EF VALUE | 50 % | ⬦ | END-DIASTOLIC AXIS LENGTH (306) | 70mm ⬦ | |
| **308B** END-DIASTOLIC VOLUME | 140 ml | ⬦ | END-SYSTOLIC AXIS LENGTH (307) | 72mm ⬦ | |
| **308C** END-SYSTOLIC VOLUME | 70 ml | ⬦ | | | |

| OUTWARD ADJUSTMENT | INWARD ADJUSTMENT | | FIX AXIS LENGTH | FIX DIAMETER LENGTH | UNIFORM DIFORMATION |
|---|---|---|---|---|---|

VOLUME / TIME

**316A** **316B** **313** **308E** **316D** **310** **316E** **311** **316H** **309**

EP 2 554 123 A1

# FIG. 4

# FIG. 5

(a)    (b)    (c)    (d)

$\Delta a_i/2$    $\Delta a_i/2$

$\Delta L$

$\Delta L$

$\Delta a_i/2$

EP 2 554 123 A1

FIG. 6

A4C

302
END-DIASTOLE

312B
306
304

303
END-SYSTOLE

312B
307
305

314
312A
312A

315

308D

| SURPASSED RE-INPUT RANGE OF EF VALUE. PLEASE CHECK. | 603 |

| | | | | | | |
|---|---|---|---|---|---|---|
| EF VALUE | 50 % | ⬦ | END-DIASTOLIC AXIS LENGTH (306) | 70mm ⬦ | RE-INPUT RANGE OF EF VALUE: ±10% | |
| END-DIASTOLIC VOLUME | 140 ml | ⬦ | END-SYSTOLIC AXIS LENGTH (307) | 72mm ⬦ | RE-INPUT RANGE OF END-DIASTOLIC VOLUME: ±20ml | |
| END-SYSTOLIC VOLUME | 70 ml | ⬦ | | | RE-INPUT RANGE OF END-SYSTOLIC VOLUME: ±20ml | |

308A
308B
308C

602

| OUTWARD ADJUSTMENT | INWARD ADJUSTMENT | | SET RE-INPUT RANGE |
|---|---|---|---|

316A     316B          601          308E

313

# FIG. 7

(a)          (b)          (c)

EP 2 554 123 A1

# FIG. 8

A4C

A2C

EP 2 554 123 A1

# FIG. 9

**301**

**A4C**

**302** END-DIASTOLE — **312B**, **306**, **304**, **314**, **312A**, **312A**

**303** END-SYSTOLE — **312B**, **307**, **305**, **315**, **312A**

**308D**

| | | | | |
|---|---|---|---|---|
| **308A** EF VALUE | 50 % ⬍ | END-DIASTOLIC AXIS LENGTH (306) | 70mm ⬍ | |
| **308B** END-DIASTOLIC VOLUME | 140 ml ⬍ | END-SYSTOLIC AXIS LENGTH (307) | 72mm ⬍ | VOLUME / TIME |
| **308C** END-SYSTOLIC VOLUME | 70 ml ⬍ | | | |

| OUTWARD ADJUSTMENT | INWARD ADJUSTMENT | | FIX AXIS LENGTH | FIX VOLUME | RE-INPUT AXIS LENGTH ERROR |
|---|---|---|---|---|---|

**313**    **308E**    **310**    **311**    **309**

**316A**  **316B**    **316E**    **316F**  **316G**

# FIG. 10

START

S101 — DISPLAY ULTRASONIC IMAGE

S201 — SET MEASUREMENT VALUE

S102 — SET MULTI-DIMENSIONAL INFORMATION

S103 — MEASUREMENT CALCULATION

S107 — RECALCULATION OF MEASUREMENT

S104 — DIPSLAY MEASRUEMENT RESULT

S106 — RE-INPUT MEASUREMENT VALUE

S105 — CHECK?

NG

OK

END

EP 2 554 123 A1

23

# FIG. 11

EP 2 554 123 A1

# FIG. 12

| | A4C | A2C | 48 % | 52 % |
|---|---|---|---|---|

| EF VALUE | 50 % | ⬍ | A4C *1 | 70mm | ⬌ |
|---|---|---|---|---|---|
| END-DIASTOLIC VOLUME | 140 ml | ⬍ | A4C *2 | 72mm | ⬌ |
| END-SYSTOLIC VOLUME | 70 ml | ⬍ | A2C *1 | 67mm | ⬌ |
| | | | A2C *2 | 69mm | ⬌ |

| OUTWARD ADJUSTMENT | INWARD ADJUSTMENT | | FIX AXIS LENGTH |
|---|---|---|---|

316A    316B

*1:END-DIASTOLIC VOLUME
*2:END-SYSTOLIC VOLUME

316D

302  306  304
303  305  307
308
314  315
311
25
EP 2 554 123 A1

# FIG. 13

301

302  306  304  306
303  307  305

52%  48%  314
52%  48%
312
315

308D

| 308 | EF VALUE | 50 % | ⬍ | END-DIASTOLIC AXIS LENGTH (306) | 70mm ⬍ | |
|---|---|---|---|---|---|---|
| | END-DIASTOLIC VOLUME | 140 ml | ⬍ | END-SYSTOLIC AXIS LENGTH (307) | 72mm ⬍ | |
| | END-SYSTOLIC VOLUME | 70 ml | ⬍ | | | |
| | OUTWARD ADJUSTMENT | INWARD ADJUSTMENT | | FIX AXIS LENGTH | FIX DIAMETER LENGTH | FIX VOLUME |

316A  316B  308E  316D  310  311  316E  316F  309

volume / time

EP 2 554 123 A1

# FIG. 14

| EF VALUE | 50 % | ⬦ |
| END-DIASTOLIC VOLUME | 140 ml | ⬦ |
| END-SYSTOLIC VOLUME | 70 ml | ⬦ |

| OUTWARD ADJUSTMENT | INWARD ADJUSTMENT | | NORMAL |

<div align="center">

**INTERNATIONAL SEARCH REPORT**

</div>

| | International application No. |
|---|---|
| | PCT/JP2011/057134 |

**A. CLASSIFICATION OF SUBJECT MATTER**

*A61B8/00*(2006.01)i, *A61B5/00*(2006.01)i

According to International Patent Classification (IPC) or to both national classification and IPC

**B. FIELDS SEARCHED**

Minimum documentation searched (classification system followed by classification symbols)
A61B8/00, A61B5/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | JP 2002-224116 A  (Matsushita Electric Industrial Co., Ltd.),<br>13 August 2002 (13.08.2002),<br>paragraphs [0035] to [0045], [0127], [0133] to [0137]<br>& US 2002/0102023 A1 | 1,2,4,7,8,<br>11,15<br>5,6,12-14<br>3,10 |
| Y | JP 2007-507248 A  (Koninklijke Philips Electronics N.V.),<br>29 March 2007 (29.03.2007),<br>paragraphs [0028] to [0033]<br>& US 2007/0016019 A1     & EP 1673013 A<br>& WO 2005/030057 A1 | 5,6,12 |

☒ Further documents are listed in the continuation of Box C.　　☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 10 May, 2011 (10.05.11) | 24 May, 2011 (24.05.11) |

| Name and mailing address of the ISA/<br>　Japanese Patent Office | Authorized officer |
|---|---|
| Facsimile No. | Telephone No. |

Form PCT/ISA/210 (second sheet) (July 2009)

## INTERNATIONAL SEARCH REPORT

| | International application No. |
|---|---|
| | PCT/JP2011/057134 |

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2004-254829 A  (Hitachi Medical Corp.), 16 September 2004 (16.09.2004), entire text; all drawings (Family: none) | 5,6 |
| Y | JP 2008-12047 A  (Aloka Co., Ltd.), 24 January 2008 (24.01.2008), entire text; all drawings & US 2008/0009735 A1    & EP 1875867 A1 | 5,6 |
| Y | JP 2007-117344 A  (Aloka System Engineering Co., Ltd.), 17 May 2007 (17.05.2007), paragraphs [0061] to [0063]; fig. 12 to 14 (Family: none) | 13 |
| Y | JP 2008-104695 A  (Toshiba Corp., Toshiba Medical Systems Corp., Toshiba Medical Systems Engineering Co., Ltd.), 08 May 2008 (08.05.2008), paragraphs [0061] to [0079], [0100] (Family: none) | 14 |

Form PCT/ISA/210 (continuation of second sheet) (July 2009)

**INTERNATIONAL SEARCH REPORT**

| International application No. |
| --- |
| PCT/JP2011/057134 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
    because they relate to subject matter not required to be searched by this Authority, namely:

2. ☒ Claims Nos.:  9
    because they relate to parts of the international application that do not comply with the prescribed requirements to such an extent that no meaningful international search can be carried out, specifically:
    The statement in claim 9 is unclear Japanese text.

3. ☐ Claims Nos.:
    because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H1099328 A
- JP 2007507248 A